(19) Europäisches Patentamt
European Patent Office
Office européen des brevets

(11) Veröffentlichungsnummer: **0 142 797**
**B1**

(12) # EUROPÄISCHE PATENTSCHRIFT

(45) Veröffentlichungstag der Patentschrift: **13.07.88**

(51) Int. Cl.⁴: **C 07 C 120/04**, C 07 C 121/70, C 07 C 121/75, C 07 D 333/24

(21) Anmeldenummer: **84113555.1**

(22) Anmeldetag: **09.11.84**

(54) **Verfahren zur Herstellung von in 2-Stellung aromatisch oder heterocyclisch substituierten Acetonitrilen.**

(30) Priorität: **15.11.83 DE 3341306**

(43) Veröffentlichungstag der Anmeldung: **29.05.85 Patentblatt 85/22**

(45) Bekanntmachung des Hinweises auf die Patenterteilung: **13.07.88 Patentblatt 88/28**

(84) Benannte Vertragsstaaten: **BE CH DE FR GB LI**

(56) Entgegenhaltungen:
**EP - A - 0 010 466**
**EP - A - 0 047 562**
**GB - A - 2 104 503**

**JOURNAL OF THE AMERICAN CHEMICAL SOCIETY, Band 68, Nr. 10, 17. Oktober 1946, Seiten 1934-1936; F.F. BLICKE et al.: "Antispasmodics. VIII"**

(73) Patentinhaber: **BASF Aktiengesellschaft, Carl-Bosch-Strasse 38, D-6700 Ludwigshafen (DE)**

(72) Erfinder: **Mueller, Josef, Dr., Rieslingstrasse 15, D-6711 Grosskarlbach (DE)**
Erfinder: **Wiersdorff, Walter-Wielant, Dr., Blockfeldstrasse 15, D-6704 Mutterstadt (DE)**
Erfinder: **Thyes, Marco, Dr., Thorwaldsenstrasse 1, D-6700 Ludwigshafen (DE)**
Erfinder: **Kirschenlohr, Werner, Dr., Alwin-Mittasch-Platz 10, D-6700 Ludwigshafen (DE)**
Erfinder: **Scherer, Hans, Dr., Plauserstrasse 16, D-6719 Weisenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 142 797 B1**

ACTORUM AG

**Beschreibung**

Die Erfindung betrifft ein Verfahren zur Herstellung von in 2-Stellung aromatisch oder heterocyclisch substituierten Acetonitrilen durch Umsetzung von aromatischen oder heterocyclischen Methylhalogeniden mit Alkalicyaniden in Gegenwart von Ketonen, Wasser und Phasentransferkatalysatoren.

Die US-PS 2 734 908 beschreibt die Umsetzung von in der Seitenkette halogensubstituierten, araliphatischen Kohlenwasserstoffen mit Alkalicyaniden in Gegenwart von Cyanide lösenden organische Lösungsmitteln, z.B. Ketonen, unter Zusatz von Alkalijodiden oder -bromiden. Diese Verfahrensweise ist jedoch nachteilig, denn wegen der langen Reaktionszeiten und geringen Konzentrationen resultieren geringe Raumzeitausbeuten. Zur Aufarbeitung muss vom Salzrückstand abfiltriert, dann das Lösungsmittel abdestilliert und der Rückstand mit einem zweiten Lösungsmittel gelöst werden. Das Gemisch wird nun getrocknet und fraktioniert destilliert. Die genannten Operationen sind technisch aufwendig und teuer.

Am Soc. 68 (1946), 1934–1936 beschreibt die Umsetzung von 2-Thienylmethylchlorid mit Natriumcyanid in einem Gemisch aus Aceton und Wasser bei 60–65°C während 4 Stunden; die Ausbeute an 2-Thienylacetonitril beträgt 81%. Diese Verfahrensweise liefert in manchen Fällen, z.B. im Falle der Umsetzung von 3-Thienylmethylchlorid, keine wesentlichen Mengen an Endstoff.

In der DE-OS 2 157 540 wird beschrieben und in einem Beispiel gezeigt, 2,5-Dichlor-3-chlormethylthiophen bevorzugt in wässrigem Isopropanol mit Natriumcyanid umzusetzen. Auch hier ist die Isolierung des Endstoffs aufwendig und es wird ein zusätzliches Lösungsmittel (Methylenchlorid) benötigt. Die Ausbeute beträgt nur 74,5%. Zur Aufarbeitung wird Isopropanol abdestilliert, der Rückstand abgekühlt und mit Methylenchlorid dreimal extrahiert. Die Extrakte werden dann abgetrennt, vereinigt, über Mangesiumsulfat getrocknet und filtriert. Das Filtergut wird dann mit Methylenchlorid gewaschen, die Waschflüssigkeit mit dem Filtrat vereinigt und das Gemisch fraktioniert destilliert.

Es wurde nun gefunden, dass man in 2-Stellung durch einen aromatischen oder heterocyclischen Rest substituierte Acetonitrile der Formel

$R^1{-}CH_2{-}CN$ I,

worin $R^1$ einen aromatischen oder heterocyclischen Rest bedeutet, durch Umsetzung von Halogenverbindungen mit Alkalicyaniden in Gegenwart von Ketonen und Wasser als Lösungsmittel vorteilhaft erhält, wenn man aromatische oder heterocyclische Methylhalogenide der Formel

$R^1{-}CH_2{-}X$ II,

worin $R^1$ die vorgenannte Bedeutung besitzt und X ein Halogenatom bezeichnet, mit Alkalicyaniden der Formel

MCN III,

worin M ein Alkaliatom bedeutet, in Gegenwart von Phasentransferkatalysatoren umsetzt.

Die Umsetzung kann für den Fall der Verwendung von Thienylmethylchlorid und Natriumcyanid durch die folgenden Formeln wiedergegeben werden:

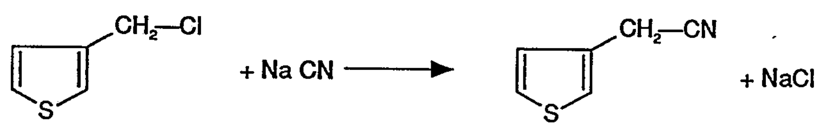


Im Vergleich zu den bekannten Verfahren liefert das Verfahren nach der Erfindung auf einfacherem und wirtschaftlicherem Wege in 2-Stellung durch einen aromatischen oder heterocyclischen Rest substituierte Acetonitrile in besserer Ausbeute und Reinheit. Die Abtrennung des Endstoffs ist einfacher und benötigt kein zusätzliches organisches Lösungsmittel. Der Gehalt an dem unerwünschten Nebenprodukt, der durch Hydrolyse des Methylhalogenids sich ergebenden Methanolverbindung, ist wesentlich geringer; die Aufarbeitung, insbesondere die fraktionierte Destillation wird dadurch in vorteilhafter Weise vereinfacht und verbilligt. Alle diese vorteilhaften Ergebnisse sind im Hinblick auf den Stand der Technik überraschend. Man hätte bei der Umsetzung in Anwesenheit von Wasser grössere Mengen Methanolverbindung als Nebenprodukt erwartet.

Die Ausgangsstoffe II und III können in stöchiometrischen Mengen oder im Überschuss jeder Komponente zur anderen, vorteilhaft in einem Verhältnis von 0,5 bis 10, insbesondere 0,9 bis 1,5 Mol Ausgangsstoff III je Mol Ausgangsstoff II umgesetzt werden. Bevorzugte Ausgangsstoffe II und dementsprechend bevorzugte Endstoffe I sind solche, in deren Formeln $R^1$ einen Arylrest mit 6 bis 14 Kohlenstoffatomen, vorteilhaft einen Phenylrest, oder einen heterocyclischen, 5- oder 6-gliedrigen Rest, der 1 oder 2 Stickstoffatome, 1 Sauerstoff- und/oder 1 Schwefelatom enthält, bedeutet, wobei der aromatische oder heterocyclische Rest noch durch inerte Gruppen oder Atome, z.B. Chloratome, Bromatome, Alkoxy- und Alkylgruppen mit jeweils zweckmässig 1 bis 8, insbesondere 1 bis 4 Kohlenstoffatomen, substituiert sein kann und X ein Bromatom oder ein Chloratom bezeichnet.

Beispielsweise sind folgende Methylhalogenide als Ausgangsstoff II geeignet: Benzylchlorid;

in 2-, 3- oder 4-Stellung durch Bromatome, Chloratome, Methyl-, Ethyl-, Propyl-, Isopropyl-, Butyl-, Isobutyl-, sec.-Butyl-, tert.-Butyl-, Methoxy-, Ethoxy-, Propoxy-, Isopropoxy-, Butoxy-, Isobutoxy-, sec.-Butoxy-, tert.-Butoxy-gruppen substituiertes Benzylchlorid; in 2,3-, 2,4-, 2,5-, 2,6-, 3,4- oder 3,5-Stellung zweifach durch vorgenannte Atome und Gruppen gleich oder verschieden substituierte Benzylchloride; in 2,3,4,-, 2,3,5-, 2,3,6-, 3,4,5-Stellung dreifach durch vorgenannte Atome und Gruppen gleich oder verschieden substituierte Benzylchloride; in 2-, 3-, 4- oder 5-Stellung durch die Chlormethylgruppe substituiertes Furan, Thiophen, Pyrrol; in 2-, 4- oder 5-Stellung durch die Chlormethylgruppe substituiertes Oxazol, Thiazol, Imidazol; in 3-, 4- oder 5-Stellung durch die Chlormethylgruppe substituiertes Isoxazol, Pyrazol; in 2-, 3-, 4-, 5- oder 6-Stellung durch die Chlormethylgruppe substituiertes Pyridin; in 3-, 4-, 5- oder 6-Stellung durch die Chlormethylgruppe substituiertes 2H-1,2-Oxazin, Pyridazin; in 2-, 4-, 5- oder 6-Stellung durch die Chlormethylgruppe substituiertes Pyrimidin, in 2-, 3-, 5- oder 6-Stellung durch die Chlormethylgruppe substituiertes Pyrazin; analog durch die Chlormethylgruppe und zusätzlich eine (eines) der vorgenannten Gruppen (Atome) substituierte Heterocyclen; entsprechende Brommethylverbindungen.

Als Alkalicyanide sind Kaliumcyanid und insbesondere Natriumcyanid bevorzugt. Als Ketone werden vorteilhaft solche der Formel

$$R^2-\overset{\overset{O}{\|}}{C}-R^3 \qquad IV,$$

worin $R^2$ und $R^3$ gleich oder verschieden sein können und jeweils einen aliphatischen, cycloaliphatischen, araliphatischen oder aromatischen Rest, zweckmässig einen Alkylrest mit 1 bis 6 Kohlenstoffatomen, einen Cycloalkylrest mit 5 bis 7 Kohlenstoffatomen, einen Aralkyl- oder Alkylarylrest mit 7 bis 12 Kohlenstoffatomen oder einen Phenylrest bedeuten, $R^2$ und $R^3$ zusammen mit der benachbarten Carbonylgruppe auch Glieder eines bevorzugt 5- bis 13-gliedrigen Ringes bezeichnen können.

Zweckmässig werden solche Ketone ausgewählt, die zusammen mit dem Wasser den Ausgangsstoff III ganz oder teilweise lösen.

Als Ketone IV kommen z.B. in Frage: Dimethyl-, Dibutyl-, Diisobutyl-, Di-sec.-butyl-, Di-tert.-butyl-, Dicyclohexyl-, Dibenzyl-, Diphenyl-keton; Methylbutyl-, Methyl-sec.-butyl-, Methyl-tert.-butyl-, Methyl-cyclohexyl-, Methylbenzyl-, Methylphenyl-keton; entsprechend unsymmetrische Ketone, die anstelle der Methylgruppe eine Ethyl-, Propyl-, Isopropyl-, Butyl-, Isobutyl-, sec.-Butyl-, tert.-Butylgruppe enthalten; Cyclopentanon, Cycloheptanon, Cyclooctanon, Cyclononanon, Cyclodecanon; bevorzugt Aceton, Methylisobutylketon, Methylethylketon, Diethylketon, Cyclohexanon, Dissopropylketon, Methylisopropylketon, Methyl-n-propylketon, Di-n-propylketon, Methylisoamylketon.

Das Keton IV wird zweckmässig in einer Menge von 20 bis 5000, insbesondere 50 bis 200 Gew.%, bezogen auf Ausgangsstoff II, das bei der Umsetzung verwendete Wasser in einer Menge von 5 bis 5000, insbesondere 30 bis 200 Gew.%, bezogen auf Ausgangsstoff II, oder Wasser in einer Menge von 5 bis 200 Gew.%, bezogen auf Keton IV, verwendet.

Die erfindungsgemässe Umsetzung wird im allgemeinen bei einer Temperatur von 20 bis 200°C, vorzugsweise von 40 bis 120°C, insbesondere von 50 bis 90°C, mit Überdruck, Unterdruck oder drucklos, kontinuierlich oder bevorzugt diskontinuierlich durchgeführt. Die Reaktionszeiten können 1 bis 20 Stunden, zweckmässig 3 bis 8 Stunden betragen.

Unter Phasentransferkatalysatoren versteht man solche Katalysatoren, die den Transport von Stoffen in binären Systemen, z.B. Wasser und organischem Lösungsmittel, verbessern. Schon kleine Mengen an Katalysator ergeben den Transfereffekt, d.h. die Ausgangsstoffe gelangen mit Hilfe kleiner Katalysatormengen von einer Phase, z.B. der wässrigen Phase, in eine andere Phase, z.B. eine bestimmte organische Lösungsmittelphase. Bezüglich der Definition, Herstellung und Eigenschaften solcher Phasentransferkatalysatoren wird auf Anorganische Chemie (1974), Seiten 187 bis 196, auf JACS, Band 93 (1971), Seiten 195 bis 199 und auf Journal of Chemical Education, Vol. 55 (1978), Seiten 429 bis 433 und 350 bis 354, verwiesen. Die Umsetzung wird in Gegenwart von Phasentransferkatalysatoren, vorteilhaft mit einer Menge von 6,5 bis 50, insbesondere von 2 bis 10 Gew.% Katalysator, bezogen auf Ausgangsstoff II, durchgeführt.

Als Phasentransferkatalysatoren kommen zweckmässig quarternäre Salze in Betracht, insbesondere Katalysatoren der Formel

$$\left[\begin{array}{ccc} & R^4 & \\ & | & \\ R^4 - & Y & - R^4 \\ & | & \\ & R^4 & \end{array}\right]^{\oplus} \quad Z^{\ominus} \qquad V,$$

worin die einzelnen Reste $R^4$ gleich oder verschieden sein können und jeweils einen aliphatischen, cycloaliphatischen, aromatischen oder araliphatischen Rest bedeuten, Y ein Stickstoffatom oder Phosphoratom bezeichnet und Z für ein Säureanion steht. Bevorzugte Katalysatoren sind solche, in deren Formeln die einzelnen Reste $R^4$ gleich oder verschieden sein können und jeweils einen Alkylrest oder einen Hydroxyalkylrest mit jeweils 1 bis 18, insbesondere 1 bis 7 Kohlenstoffatomen, einen Cycloalkylrest mit 5 bis

8 Kohlenstoffatomen, einen Aralkylrest oder Alkylarylrest mit 7 bis 12 Kohlenstoffatomen oder einen Phenylrest bedeuten.

Es kommen z.B. als Phasentransferkatalysatoren V in Frage: Tetramethyl-, Teträthyl-, Tetra-n-propyl-, Tetraisopropyl-, Tetra-n-butyl-, Tetraisobutyl-, Tetra-sec.-butyl-, Tetra-tert.-butyl-, Tetrapentyl-, Tetrahexyl-, Tetra-n-heptyl-, Tetraoctyl-, Tetranonyl-, Tetradecyl-, Tetraundecyl-, Tetradodecylammoniumchlorid; (Tetra-β-hydroxyethyl)-ammoniumchlorid, (Tetra-ω-hydroxypropyl)-ammoniumchlorid, (Tetra-β-hydroxypropyl)-ammonium-chlorid, (Tetra-δ-hydroxybutyl)-ammoniumchlorid, (Tetra-ω-hydroxybutyl)-ammoniumchlorid, (Tetra-β-hydroxybutyl)-ammoniumchlorid, Tetra-(α,α-dimethyl-β-hydroxyethyl)-ammoniumchlorid, Tetra-(α-methyl-β-hydroxypropyl)-ammoniumchlorid, Tetra-(β,β-dimethyl-β-hydroxyethyl)-ammoniumchlorid, Tetra-(α-ethyl-β-hydroxyethyl)-ammoniumchlorid, Tetra-(α-methyl-ω-hydroxy-propyl)-ammoniumchlorid, Tetra-(β-methyl-ω-hydroxypropyl)-ammoniumchlorid, (Tetra-β-hydroxypentyl)-ammoniumchlorid, (Tetra-ω-hydroxypentyl)-ammoniumchlorid, (Tetra-δ-hydroxypentyl)-ammoniumchlorid; durch quartäre Substituierung mit vorgenannten Substituenten und/oder mit der Phenyl-, Benzyl-, Cyclohexyl-, Toluyl-, Methylcyclohexyl-, Phenylethyl-, Phenylpropyl-, Phenylbutylgruppe am Stickstoffatom sich entsprechend aus Anilin, Benzylamin, o,m,p-Toluidin, Triphenyl-, Tribenzyl-, Tricyclo-hexyl-, Tri(methylcyclohexyl)-, Tri(phenylethyl)-, Tri(phenylpropyl)-, Tri(phenylbutyl)-amin, in 2-, 3-, 4-Stellung einfach oder in 2,4-, 2,3-, 2,6-, 2,5-, 3,4-, 2,5-Stellung zweifach durch die Methylgruppe an jedem Phenylring substituiertem Triphenylamin ergebende Ammoniumchloride, die auch Ammoniumchlorid mit 4 vorgenannten aber völlig oder teilweise unterschiedlichen Resten sein können, z.B. die sich durch Substituierung mit dem Methylrest aus N,N-Dimethylanilin, N-Methyl-N,N-diethylamin, N,N-Dicyclohexyl-N-methylamin, N-Methyl-N-ethyl-N-n-progylamin, ergebenden quartären Ammoniumchloride oder z.B. Dimethylbenzyldodecyl-, Cetyltrimethyl-, Methyltriethyl-, Dimethyldiphenyl-, Trimethyl-(o-tert.-butylphenyl)-, Triethyldodecyl-, Trimethyl-tridecyl-, Trimethyl-diphenylmethyl-, Trimethyl-dodecyl-, Trimethyl-β-hydroxyethyl, n-Propyl-trimethyl-, Isoamyltrimethyl-, Benzyl-dimethyl-n-octyl-, Benzyl-trimethyl-, Benzyl-triethyl-, Phenyl-trimethyl-, Dimethyl-dodecyl-phenyl-, Trimethyl-(phenyl-(1)-ethyl)-, Trimethyl-(phenyl-(2)-ethyl)-ammoniumchlorid; entsprechende Ammoniumbromide; homolge quartäre Ammoniumsalze von anorganischen oder organischen einbasischen oder mehrbasischen Säuren wie Chlorwasserstoff, Bromwasserstoff, Jodwasserstoff, Perchlorsäure, Schwefelsäure, Phosphorsäure, salpetrige Säure, Salpetersäure, Kohlensäure; Sulfonsäuren wie Benzol- und p-Toluolsulfonsäure; Bor enthaltenden Säuren wie Borsäure, Borfluorwasserstoffsäure; oder entsprechende Gemische. Bevorzugt sind die quartären Ammoniumsalze der Salzsäure, Bromwasserstoffsäure, Schwefelsäure, Phosphorsäure mit vorgenannten Substituenten am Heteroatom und/oder Säureanionen sowie homologe Phosphoniumsalze.

Die Reaktion kann wie folgt durchgeführt werden: Ein Gemisch von Keton IV, Ausgangsstoff II und III, quartärem Salz V und Wasser wird während der Reaktionszeit bei der Reaktionstemperatur gehalten. Dann wird aus dem Reaktionsgemisch der Endstoff in üblicher Weise, z.B. durch Versetzen mit Wasser, Phasentrennung und Destillation, abgetrennt.

Die nach dem Verfahren der Erfindung herstellbaren, in 2-Stellung aromatisch oder heterocyclisch substituierten Acetonitrile, sind wertvolle Ausgangsstoffe für die Herstellung von Pharmazeutika, Farbstoffen und Schädlingsbekämpfungsmitteln. Bezüglich der Verwendung wird auf die vorgenannten Veröffentlichungen verwiesen.

Beispiel 1

Thienyl-3-acetonitril

In einem 2 l-Rührgefäss wurden 98 g (2 mol) NaCN, 230 ml Wasser, 20 ml 50 gewichtsprozentiger Tetrabutylammoniumbromidlösung und 300 ml Methylisobutylketon vorgelegt. Das Gemisch wurde auf 60°C erhitzt. Innerhalb einer Stunde wurden zwischen 60 und 70°C 225 g Thienyl-3-methylchlorid zugeführt. Nach beendeter Zugabe wurde das Gemisch noch 6 Stunden bei 70°C nachgerührt, dann mit 200 ml Wasser versetzt und die untere wässrige Phase abgetrennt. Die Destillation der organischen Phase lieferte 193 g Thienyl-3-acetonitril, das als Nebenprodukt nur 0,4 Gew.% Thienyl-3-methanol enthielt. ($KP_{30\ mbar}$: 134–136°C, Ausbeute 91,8% der Theorie.)

Beispiel 2 (Vergleich)

Thienyl-3-acetonitril

In einem 2 l-Rührgefäss wurden 500 ml Aceton, 98 g (2 mol) NaCN und 2 g NaJ vorgelegt. Unter Rückfluss wurden dann 225 g Thienyl-3-methylchlorid innerhalb 1 Stunde zugefügt und anschliessend 42 Stunden unter Rückfluss gekocht. Dann wurden 500 ml Wasser zugegeben und nach Abtrennung der wässrigen Phase die organische Phase destilliert.

Ausbeute an Endstoff: 186,0 g Thienyl-3-acetonitril (89,3% der Theorie), $Kp_{30\ mbar}$: 131-132°C.

Beispiel 3 (Vergleich)

Thienyl-3-acetonitril

In einem 2 l-Rührgefäss wurden 450 ml Ethanol, 50 ml $H_2O$, 2 g NaJ, 98 g (2 mol) NaCN vorgelegt und unter Rückflusstemperatur 225 g Thienyl-3-methylchlorid innerhalb von 30 Minuten zugetropft. Nach beendeter Zugabe wurde das Gemisch noch 5 Stunden nachgerührt, dann wurden 250 ml Ethanol abdestilliert und 400 ml Wasser zugeführt. Die untere wässrige Phase wurde zweimal mit 150 ml Essigester extrahiert und die vereinigten organischen Phasen destilliert.

Ausbeute an Endstoff: 152,1 g (72,7% der Theorie), $Kp_{30\ mbar}$: 130–132°C.

Beispiel 4

Thienyl-3-acetonitril

Es wurden 75 g NaCN, 56 g Methylisobutylketon, 13,7 g einer 50 gewichtsprozentigen, wässrigen Lösung von N-Benzyl-N,N,N-trimethyl-ammoniumchlorid und 28 g Wasser vorgelegt. Unter Rühren wurde bei 68–72°C eine Mischung aus 164 g Thienyl-3-methylchlorid und 100 g Methylisobutylketon während 0,5 Stunden zugetropft. Nach Zugabe wurde das Gemisch 3 Stunden bei 70°C gerührt, weitere 28 g Wasser wurden zugefügt und noch 3 Stunden bei 70–75°C nachgerührt. Dann wurden 200 ml Wasser zugesetzt und die untere wässrige Phase abgetrennt. Die Destillation der organischen Phase ergab 141 g Thienyl-3-acetonitril (92,6% der Theorie), $Kp_{30\ mbar}$: 134–136°C.

Beispiel 5 (Vergleich)

Thienyl-3-scetonitril

Eine Mischung aus 133 g Thienyl-3-methylchlorid, 60 ml Aceton und 74 g Natriumcyanid wurde in 100 ml Wasser gelöst und bei 60–65°C 4 Stunden gerührt. Dann wurden 200 cm Wasser zugefügt, die organische Phase abgetrennt und destilliert.

1. Fraktion: 87 g, $Kp_{30\ mbar}$: 80–82°C (Thienyl-3-methylchlorid)
2. Fraktion: 20 g, $Kp_{30\ mbar}$: 134–136°C (Thienyl-3-acetonitril).

Ausbeute an Thienyl-3-acetonitril: 16,3% der Theorie.

Beispiel 6

4-Methoxybenzylcyanid

Eine Lösung aus 91,1 g (1,86 mol) Natriumcyanid, 50 ml Wasser, 27,8 ml einer 40 gewichtsprozentigen Lösung von N-Benzyl-N,N,N-trimethylammoniumchlorid und 280 ml Methylisobutylketon wurde vorgelegt und bei 55°C wurden unter Rühren innerhalb von 1 Stunde 245 g 4-Methoxybenzylchlorid zugetropft. Dann wurde das Gemisch 3 Stunden bei 65°C nachgerührt und mit 400 ml Wasser verdünnt. Nach Abtrennung der organischen Phase wurde das Lösungsmittel verdampft und der verbleibende Rückstand destilliert.

Ausbeute an Endstoff: 207 g (90% der Theorie); $Kp_{1\ mbar}$: 120°C.

Beispiel 7

Benzylcyanid

Eine Lösung aus 49 g (1,1 mol) Natriumcyanid, 50 ml $H_2O$, 15 g N-Benzyl-N,N,N-trimethyl-ammoniumchlorid und 150 ml Methylisobutylketon wurde auf 70°C erhitzt und unter Rühren wurden innerhalb von 30 Minuten 126,5 g (1 mol) Benzylchlorid zugetropft. Dann wurde das Gemisch 3 Stunden bei 65–70°C nachgerührt, mit 150 ml $H_2O$ verdünnt, die organische Phase abgetrennt und destilliert.

Ausbeute an Endstoff: 112 g (95,7% der Theorie), $Kp_{1\ mbar}$: 75°C.

**Patentansprüche**

Verfahren zur Herstellung von in 2-Stellung durch einen aromatischen oder heterocyclischen Rest substituierten Acetonitrilen der Formel

$R^1$-$CH_2$ I,

worin $R^1$ einen aromatischen oder heterocyclischen Rest bedeutet, durch Umsetzung von Halogenverbindungen mit Alkalicyaniden in Gegenwart von Ketonen und Wasser als Lösungsmittel, dadurch gekennzeichnet, dass man aromatische oder heterocyclische Methylhalogenide der Formel

$R^1$-$CH_2$-X II,

worin $R^1$ die vorgenannte Bedeutung besitzt und X ein Halogenatom bezeichnet, mit Alkalicyaniden der Formel

MCN III,

worin M ein Alkaliatom bedeutet, in Gegenwart von Phasentransferkatalysatoren umsetzt.

**Revendications**

Procédé de préparation d'acétonitriles substitués en position 2 par un radical aromatique ou hétérocyclique, de formule

$R^1$-$CH_2$-C I,

dans laquelle $R^1$ représente un radical aromatique ou hétérocyclique, par réaction de composés halogénés avec des cyanures alcalins en présence de cétones et d'eau servant de solvant, caractérisé en ce qu'on fait réagir, en présence de catalyseurs de transfert de phase, des halogénures de méthyle aromatiques ou hétérocycliques de formule

$R^1$-$CH_2$-X II,

dans laquelle $R^1$ a la signification donnés ci-dessus et X représente un atome d'halogène, avec des cyanures alcalins de formule

MCN III,

dans laquelle M représente un atome de métal alcalin.

**Claims**

A process for the preparation of an acetonitrile which is substituted in the 2-position by an aromatic or heterocyclic radical and is of the formula

$R^1$-$CH_2$-CN I,

where $R^1$ is an aromatic or heterocyclic radical, by reacting a halogen compound with an alkali metal cyanide in the presence of a ketone and water as solvents, wherein an aromatic or heterocyclic methyl halide of the formula

$$R^1\text{-}CH_2\text{-}X \qquad II,$$

where $R^1$ has the abovementioned meaning and X is a halogen atom, is reacted with an alkali metal cyanide of the formula

$$MCN \qquad III,$$

where M is an alkali metal atom, in the presence of a phase transfer catalyst.